# EUROPEAN PATENT APPLICATION

(11) **EP 4 516 815 A1**
(43) Date of publication of application: **05.03.2025**
(21) Application number: 23796304.6
(22) Date of filing: 24.04.2023
(51) Int. Cl.: C08F 2/44, C08F 2/00, C08F 20/00

(54) **COMPOSITION**

(30) Priority: 27.04.2022 JP 2022073191
(71) Applicant: Sumitomo Chemical Company, Limited, Tokyo 103-6020 (JP)
(72) Inventor: YASUTOMI, Yoichi, Niihama-shi, Ehime 792-8521 (JP); KADOYAI, Hidenori, Niihama-shi, Ehime 792-8521 (JP)
(74) Representative: Global IP Europe Patentanwaltskanzlei
(86) International application number: PCT/JP2023/016063
(87) International publication number: WO 2023/210564

(57) **Abstract**

The present invention improves the polymerization rate. The present invention provides a composition which contains methyl methacrylate, methyl isobutyrate and methyl propionate, wherein: the content of the methyl methacrylate is 90% by mass or more; and at least one requirement that is selected from the group consisting of the requirements (1) and (2) described below is satisfied. Requirement (1): The concentration of the methyl isobutyrate is from 20 ppm by mass to 3,000 ppm by mass, and the concentration of the methyl propionate is from 250 ppm by mass to 3,000 ppm by mass. Requirement (2): The concentration of the methyl isobutyrate is from 350 ppm by mass to 3,000 ppm by mass, and the concentration of the methyl propionate is from 5 ppm by mass to 250 ppm by mass.

## Description

### TECHNICAL FIELD

The present invention relates to a composition, and relates to a composition containing methyl methacrylate as a main component.

### BACKGROUND ART

Polymethyl methacrylate (PMMA), which is a polymer obtained by polymerizing methyl methacrylate, is excellent in transparency and also in weather resistance. Therefore, polymethyl methacrylate is widely used as a material for members constituting automobile parts, signages, display devices, and the like.

Typical examples of a method of producing methyl methacrylate as a raw material of polymethyl methacrylate include "acetone cyanohydrin (ACH) method" in which acetone and hydrogen cyanide (hydrocyanic acid) are used as raw materials, "C4 direct oxidation method" in which isobutylene or tert-butyl alcohol is used as a raw material, and "alpha method" in which ethylene is used as a raw material.

Here, in any of these methods of producing methyl methacrylate, the produced reaction product (methyl methacrylate) inevitably contains impurities such as by-products.

Further, there is known a technique for simply suppressing deterioration of properties such as an increase in yellowness and a decrease in light transmittance caused by such impurities at low cost by devising the composition even when the impurities are inevitably contained (see Patent Document 1).

### PRIOR ART DOCUMENT

### PATENT DOCUMENT

Patent Document 1: JP-B-6889322

### SUMMARY OF THE INVENTION

### PROBLEMS TO BE SOLVED BY THE INVENTION

However, according to Patent Document 1 described above, it is certainly possible to easily obtain a composition containing methyl methacrylate as a main component, which has properties such as excellent heat resistance at low cost, but a polymerization rate when the composition is polymerized may not be sufficient.

### MEANS FOR SOLVING THE PROBLEMS

As a result of intensive studies to solve the above problems, the present inventors have found that the above problems can be solved by adjusting a content of a predetermined component that can be contained in a composition, thereby completing the present invention.

That is, the present invention provides the following [1] to [12].
[1] A composition containing methyl methacrylate, methyl isobutyrate, and methyl propionate,
   in which a content of the methyl methacrylate is 90% by mass or more, and
   at least one requirement that is selected from a group consisting of the following requirements (1) and (2) is satisfied,
   Requirement (1): a concentration of the methyl isobutyrate is from 20 ppm by mass to 3,000 ppm by mass, and a concentration of the methyl propionate is from 250 ppm by mass to 3,000 ppm by mass,
   Requirement (2): a concentration of the methyl isobutyrate is from 350 ppm by mass to 3,000 ppm by mass, and a concentration of the methyl propionate is from 5 ppm by mass to 250 ppm by mass.
[2] The composition according to [1], in which the content of the methyl methacrylate is 95% by mass or more.
[3] The composition according to [1] or [2], in which the content of the methyl methacrylate is 98% by mass or more.
[4] The composition according to any one of [1] to [3], in which the requirement (1) is satisfied.
[5] The composition according to any one of [1] to [4], in which the concentration of the methyl isobutyrate is from 20 ppm by mass to 2,500 ppm by mass.
[6] The composition according to any one of [1] to [5], in which the concentration of the methyl propionate is from 250 ppm by mass to 2,500 ppm by mass.
[7] The composition according to any one of [1] to [6], in which a ratio of the concentration of the methyl propionate to the concentration of the methyl isobutyrate is from 0.1 to 2.0.
[8] The composition according to any one of [1] to [7], further containing
   methyl acrylate,
   in which a concentration of the methyl acrylate is from 5 ppm by mass to 50,000 ppm by mass.
[9] The composition according to any one of [1] to [8], in which
   one or two or more selected from a group consisting of methyl methacrylate, methyl isobutyrate, and methyl propionate include a component regenerated from polymethyl methacrylate.
[10] The composition according to any one of [1] to [9], further containing
   one or two or more selected from a group consisting of methanol, methyl 2,4-dimethyl-4-pentenoate, 1-butanol, and butyl acrylate,
   in which a concentration of each of the methanol, methyl 2,4-dimethyl-4-pentenoate, 1-butanol, and butyl acrylate is from 10 ppm by mass to 5,000 ppm by mass.
[11] The composition according to any one of [1] to [10], further containing
   one or two or more selected from a group consisting of methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene,
   in which a concentration of each of the methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene is from 10 ppm by mass to 1,000 ppm by mass.
[12] A method of producing a polymer, the method including polymerizing the composition according to any one of [1] to [11].

### EFFECT OF THE INVENTION

According to the present invention, it is possible to provide a composition having an excellent polymerization rate particularly when the composition is polymerized.

### MODE FOR CARRYING OUT THE INVENTION

Hereinafter, embodiments of the present invention will be described in detail. The present invention is not limited by the following description, and each element according to the embodiments of the present invention can be appropriately changed without departing from the gist of the present invention.

### 1. Composition

A composition according to the present embodiment is a composition containing methyl methacrylate, methyl isobutyrate, and methyl propionate,
in which a content of the methyl methacrylate is 90% by mass or more, and
at least one requirement that is selected from a group consisting of the following requirements (1) and (2) is satisfied.
Requirement (1): a concentration of the methyl isobutyrate is from 20 ppm by mass to 3,000 ppm by mass, and a concentration of the methyl propionate is from 250 ppm by mass to 3,000 ppm by mass.
Requirement (2): a concentration of the methyl isobutyrate is from 350 ppm by mass to 3,000 ppm by mass, and a concentration of the methyl propionate is from 5 ppm by mass to 250 ppm by mass.

According to the composition containing methyl methacrylate as a main component of the present embodiment, as long as any one or both of the requirements (1) and (2) are satisfied, even when a substance that does not contribute to the production of polymethyl methacrylate (polymerization of methyl methacrylate) such as methyl isobutyrate, methyl propionate, or methyl acrylate is contained, a residual amount of methyl methacrylate after polymerization can be effectively reduced and the polymerization rate can be effectively improved without intentionally applying a strict purification method that is conventionally considered to be essential.

The composition of the present embodiment is usually in the form of a solution, but is not limited thereto. Hereinafter, components that may be contained in the composition of the present embodiment will be described in detail.

### (1) Methyl Methacrylate

"Methyl methacrylate" herein refers to methyl methacrylate that is substantially free of impurities such as by-products such as methyl isobutyrate. However, the present invention is not limited to the definition on the proviso that the object of the present invention is not impaired. In other words, "methyl methacrylate" may contain impurities that cannot be removed by an ordinary purification method or may contain impurities in a content that cannot be detected by an ordinary detection method.

A method of producing methyl methacrylate to be used as a raw material of the composition of the present embodiment is not particularly limited. Examples of the method of producing methyl methacrylate as a raw material of the present embodiment include the methods described above: ACH method, C4 direct oxidation method, and alpha method. Methyl methacrylate as a raw material may be produced by any of these methods.

As a raw material of the composition of the present embodiment, in addition to methyl methacrylate produced by the above production method, it is possible to use methyl isobutyrate or a product containing methyl isobutyrate and methyl acrylate (hereinafter, referred to as "methyl methacrylate product") in a content that is mixed inevitably in the composition depending on the production method.

Methyl methacrylate (or methyl methacrylate product) that is to be used as a raw material of the composition of the present embodiment and is produced by the above production method may be purified by any conventionally-known suitable purification method.

A content of methyl isobutyrate that may be contained in the methyl methacrylate product as a raw material of the composition of the present embodiment is more than 0 ppm by mass and 300 ppm by mass or less, and/or a content of methyl acrylate that may be contained in the methyl methacrylate product is from 0 ppm by mass to 200 ppm by mass, when the total amount of the methyl methacrylate product is 100 parts by mass.

As methyl methacrylate as a raw material of the composition of the present embodiment, methyl methacrylate (may be referred to as "regenerated methyl methacrylate") regenerated from polymethyl methacrylate by so-called chemical recycling may be used.

In the present embodiment, regenerated methyl methacrylate is polymethyl methacrylate (PMMA) obtained by polymerizing methyl methacrylate by being subjected to any conventionally-known suitable injection molding step or the like, and more specifically, is methyl methacrylate obtained, for example, by thermally decomposing (depolymerizing) polymethyl methacrylate (PMMA) derived from a molded product containing, as a main component, polymethyl methacrylate (PMMA) that is distributed on the market and recovered, by heat treatment under any conventionally-known suitable conditions.

According to the chemical recycling described above, regenerated methyl methacrylate can be recovered with a high yield. In addition, any conventionally-known suitable purification step is performed after the depolymerization, such that it is possible to reduce a content (concentration) of low-boiling-point compounds (impurities) that are by-products, for example, methyl propionate, methyl isobutyrate, and methyl acrylate, that can be inevitably contained in a product containing methyl methacrylate as a main component produced by the depolymerization.

In the composition of the present embodiment, one or two or more selected from a group consisting of methyl methacrylate, methyl isobutyrate, and methyl propionate may include a component regenerated from polymethyl methacrylate. That is, methyl isobutyrate and/or methyl propionate, which is a component that can be inevitably contained in a product produced from polymethyl methacrylate by depolymerization, can be used as it is as a component of the composition of the present embodiment without being removed by purification.

In the composition of the present embodiment, the content of the methyl methacrylate is usually 85% by mass or more, preferably 90% by mass or more, more preferably 95% by mass or more, still more preferably 98% by mass or more, and particularly preferably 99% by mass or more, when the total mass of the composition is 100% by mass, from the viewpoint of improving the polymerization rate when the composition is polymerized.

### (2) Methyl Isobutyrate

As methyl isobutyrate, in addition to methyl isobutyrate that is produced by the method of producing methyl methacrylate described above and is mixed inevitably in the composition, and methyl isobutyrate that is inevitably produced by depolymerization in the chemical recycling described above, it is possible to use methyl isobutyrate separately produced by any conventionally-known suitable production method particularly from the viewpoint of adjusting a content of the methyl isobutyrate in the composition.

Examples of the method of producing methyl isobutyrate include esterification of isobutyramide to obtain methyl isobutyrate, methoxy-carbonylation of propylene to obtain methyl isobutyrate, and methyl-esterification of isobutyric acid to obtain methyl isobutyrate.

"Methyl isobutyrate" herein refers to methyl isobutyrate substantially free of impurities such as by-products. However, the present invention is not limited thereto provided that the object of the present invention is not impaired. In other words, particularly, "methyl isobutyrate" to be added to the composition may contain impurities that cannot be removed by an ordinary purification method or may contain impurities in a content that cannot be detected by an ordinary detection method.

As the methyl isobutyrate, methyl isobutyrate commercially available as a reagent or the like can be used.

In the composition of the present embodiment, when the total amount of the composition is 100 parts by mass, a concentration of the methyl isobutyrate is from 20 ppm by mass to 3,000 ppm by mass in the case of the requirement (1), and is from 350 ppm by mass to 3,000 ppm by mass in the case of the requirement (2).

In the composition of the present embodiment, the concentration of the methyl isobutyrate is preferably from 20 ppm by mass to 2,500 ppm by mass and more preferably from 20 ppm by mass to 2,000 ppm by mass in the case of the requirement (1), and is preferably from 350 ppm by mass to 2,500 ppm by mass and more preferably from 350 ppm by mass to 2,000 ppm by mass in the case of the requirement (2).

According to the composition of the present embodiment, even when methyl isobutyrate is further contained at the above concentration, the polymerization rate when the composition is polymerized can be further improved. Note that, in the requirements (1) and (2), when the concentration of the methyl isobutyrate is more than 3,000 ppm by mass, the polymerization rate tends to decrease when the composition is polymerized.

### (3) Methyl Propionate

The composition of the present embodiment may contain methyl propionate.

As methyl propionate, in addition to methyl propionate that is inevitably produced by the method of producing methyl methacrylate described above, and methyl propionate that is inevitably produced by depolymerization in the chemical recycling described above, it is possible to use methyl propionate separately produced by any conventionally-known suitable production method.

Examples of the method of producing methyl propionate include methoxy-carbonylation of ethylene to obtain methyl propionate and methyl-esterification of propionic acid to obtain methyl propionate.

As methyl propionate, methyl propionate commercially available as a reagent or the like can be used.

"Methyl propionate" herein refers to methyl propionate substantially free of impurities such as by-products. However, the present invention is not limited thereto provided that the object of the present invention is not impaired. In other words, particularly, "methyl propionate" to be added to the composition may contain impurities that cannot be removed by an ordinary purification method or may contain impurities in a content that cannot be detected by an ordinary detection method.

A concentration of the methyl propionate that can be contained in the composition of the present embodiment is from 250 ppm by mass to 3,000 ppm by mass in the case of the requirement (1), and is from 5 ppm by mass to 250 ppm by mass in the case of the requirement (2), when the total amount of the composition is 100 parts by mass.

In the composition of the present embodiment, the concentration of the methyl propionate is preferably from 250 ppm by mass to 2,500 ppm by mass and more preferably from 250 ppm by mass to 2,000 ppm by mass in the case of the requirement (1), and is preferably from 5 ppm by mass to 250 ppm by mass and more preferably from 5 ppm by mass to 200 ppm by mass in the case of the requirement (2).

According to the composition of the present embodiment, even when methyl propionate is further contained at the above concentration, the polymerization rate when the composition is polymerized can be further improved. Note that, in the requirement (1), when the concentration of the methyl isobutyrate is more than 3,000 ppm by mass, the polymerization rate tends to decrease when the composition is polymerized.

### (4) Methyl Acrylate

As methyl acrylate, in addition to methyl acrylate that is produced by the method of producing methyl methacrylate described above and is mixed inevitably in the composition, and methyl acrylate that is inevitably produced by depolymerization in the chemical recycling described above, it is possible to use methyl acrylate separately produced by any conventionally-known suitable production method.

Examples of the method of producing methyl acrylate include methoxy-carbonylation of acetylene to obtain methyl acrylate, alcoholysis of acrylonitrile to obtain methyl acrylate, and methyl-esterification of acrylic acid to obtain methyl acrylate.

It is possible to use methyl acrylate commercially available as a reagent or the like.

"Methyl acrylate" herein refers to methyl acrylate substantially free of impurities such as by-products. However, the present invention is not limited thereto provided that the object of the present invention is not impaired. In other words, particularly, "methyl acrylate" to be added to the composition may contain impurities that cannot be removed by an ordinary purification method or may contain impurities in a content that cannot be detected by an ordinary detection method.

The concentration of methyl acrylate that can be contained in the composition of the present embodiment is preferably from 5 ppm by mass to 50,000 ppm by mass and more preferably from 5 ppm by mass to 30,000 ppm by mass.

According to the composition of the present embodiment, even when methyl acrylate is further contained at the above concentration, the polymerization rate when the composition is polymerized can be further improved.

### (5) Other Low-Content Components

In particular, when regenerated methyl methacrylate is used as methyl methacrylate, the composition of the present embodiment may contain, as other low-content components, for example, methanol, methyl 2,4-dimethyl-4-pentenoate, 1-butanol, and butyl acrylate, and may further contain methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene.

When the composition of the present embodiment further contains one or two or more selected from a group consisting of methanol, methyl 2,4-dimethyl-4-pentenoate, 1-butanol, and butyl acrylate among the low-content components, a concentration of each of the methanol, methyl 2,4-dimethyl-4-pentenoate, 1-butanol, and butyl acrylate is preferably from 10 ppm by mass to 5,000 ppm by mass, and more preferably from 10 ppm by mass to 4,000 ppm by mass, when the total amount of the composition is 100 parts by mass.

When the composition of the present embodiment further contains one or two or more selected from a group consisting of methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene among the low-content components, a concentration of each of the methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene is preferably from 10 ppm by mass to 1,000 ppm by mass, and more preferably from 10 ppm by mass to 800 ppm by mass, when the total amount of the composition is 100 parts by mass.

According to the composition of the present embodiment, even when the low-content components are further contained at the above concentration, the polymerization rate when the composition is polymerized can be further improved.

### (6) Other Components

On the proviso that the object of the present invention is not impaired, in addition to the methyl methacrylate, methyl isobutyrate, methyl acrylate, and methyl propionate described above, the composition of the present embodiment may contain other components such as a release agent, a polymerization modifier, a polymerization initiator, an ultraviolet absorber, and a colorant that may be added to form a molded body having predetermined properties. Hereinafter, other components that can be contained in the composition of the present embodiment will be described.

### (i) Release Agent

In production of polymethyl methacrylate using the composition of the present embodiment and production of a molded body thereof, the composition of the present embodiment may particularly contain a release agent for improving releasability of the molded body.

Examples of the release agent that may be contained in the composition of the present embodiment include a higher fatty acid ester, a higher aliphatic alcohol, a higher fatty acid, a higher fatty acid amide, a higher fatty acid metal salt, and a fatty acid derivative.

Specific examples of the release agent that may be contained in the composition of the present embodiment include sodium di-(2-ethylhexyl) sulfosuccinate, stearyl alcohol, methyl stearate, and stearic acid amide. In the composition of the present embodiment, these exemplified release agents may be used alone or in combination of two or more kinds thereof.

A content of the release agent in the composition of the present embodiment is, for example, 0.01 parts by mass to 1.0 part by mass when the total amount of the composition is 100 parts by mass.

### (ii) Polymerization Modifier

The composition of the present embodiment may contain a polymerization modifier that can adjust a polymerization rate in a polymerization reaction.

As the polymerization modifier that may be contained in the composition of the present embodiment, it is possible to use any conventionally-known suitable polymerization modifier. Examples of the polymerization modifier include compounds that can adjust a polymerization rate so as to reduce the polymerization rate.

Specific preferred examples of the polymerization modifier include mercaptan compounds such as n-butyl mercaptan and n-octyl mercaptan; terpenoid compounds such as limonene, myrcene, α-terpinene, β-terpinene, γ-terpinene, terpinolene, β-pinene, and α-pinene; and an α-methylstyrene dimer. In the present embodiment, as the polymerization modifier, terpinolene, which is a terpenoid compound, is preferably used.

In the composition of the present embodiment, these exemplified polymerization modifiers may be used alone or in combination of two or more kinds thereof.

A content of the polymerization modifier in the composition of the present embodiment is, for example, 0.001 parts by mass to 0.5 parts by mass when the total amount of the composition is 100 parts by mass.

### (iii) Polymerization Initiator

Examples of the polymerization initiator that may be contained in the composition of the present embodiment include a radical polymerization initiator, a diacyl peroxide initiator, a dialkyl peroxide initiator, a peroxyester initiator, a percarbonate initiator, and a peroxyketal initiator.

Specific examples of the radical polymerization initiator include azo compounds such as 1,1'-azobis(cyclohexane-1-carbonitrile), 2,2'-azobis(2,4,4-trimethylpentene), 2,2'-azobis(2-methylpropane), 2-cyano-2-propylazoformamide, 2,2'-azobis(2-hydroxy-methylpropionate), 2,2'-azobis(2-methyl-butyronitrile), 2,2'-azobisisobutyronitrile, 2,2'-azobis[2-(2-imidazolin-2-yl)propane], and dimethyl 2,2'-azobis(2-methyl propionate).

Specific examples of the diacyl peroxide initiator and the dialkyl peroxide initiator include dicumyl peroxide, tert-butyl cumyl peroxide, di-tert-butyl peroxide, benzoyl peroxide, and lauroyl peroxide.

Specific examples of the peroxyester initiator include tert-butyl peroxy-3,3,5-trimethylhexanoate, tert-butyl peroxylaurate, tert-butyl peroxyisobutyrate, tert-butyl peroxyacetate, di-tert-butyl peroxyhexahydroterephthalate, di-tert-butyl peroxyazelate, tert-butyl peroxy-2-ethylhexanoate, 1,1,3,3-tetramethylbutyl peroxy-2-ethylhexanoate, and tert-amylperoxy-2-ethylhexanoate.

Specific examples of the percarbonate initiator include tert-butylperoxyallyl carbonate and tert-butylperoxyisopropyl carbonate.

Specific examples of the peroxyketal initiator include 1,1-di-tert-butylperoxycyclohexane, 1,1-di-tert-butylperoxy-3,3,5-trimethylcyclohexane, and 1,1-di-tert-hexylperoxy-3,3,5-trimethylcyclohexane.

In the composition of the present embodiment, these exemplified polymerization initiators may be used alone or in combination of two or more kinds thereof.

A content of the polymerization initiator in the composition of the present embodiment is, for example, 0.01 parts by mass to 5 parts by mass when the total amount of the composition is 100 parts by mass.

### (iv) Ultraviolet Absorber

The composition of the present embodiment may contain an ultraviolet absorber in order to further improve weather resistance of the polymethyl methacrylate to be produced and a molded body thereof.

Preferred examples of the ultraviolet absorber that may be contained in the composition of the present embodiment include a benzophenone ultraviolet absorber, a cyanoacrylate ultraviolet absorber, a benzotriazole ultraviolet absorber, a malonic acid ester ultraviolet absorber, and an oxalanilide ultraviolet absorber.

Specific examples of the ultraviolet absorber include 2-(2'-hydroxy-5'-methylphenyl)benzotriazole, 2-hydroxy-4-n-octylbenzophenone, 2-(3-tert-butyl-2-hydroxy-5-methylphenyl)-5-chlorobenzotriazole, 2-(2-hydroxy-3,5-di-tert-pentylphenyl)benzotriazole, and 2,4-di-tert-butylphenyl-3,5-di-tert-butyl-4-hydroxybenzoate.

In the composition of the present embodiment, these exemplified ultraviolet absorbers may be used alone or in combination of two or more kinds thereof.

A content of the ultraviolet absorber in the composition of the present embodiment is, for example, 0.001 parts by mass to 1 part by mass when the total amount of the composition is 100 parts by mass.

### (v) Colorant

The composition of the present embodiment may contain a colorant in order to turn polymethyl methacrylate to be produced and a molded body thereof into any suitable color.

Preferred examples of the colorant that may be contained in the composition of the present embodiment include a perylene dye, a perinone dye, a pyrazolone dye, a methine dye, a coumarin dye, a quinophthalone dye, a quinoline dye, an anthraquinone dye (for example: Sumiplast Violet B), an anthraquinone dye, an anthrapyridone dye, a thioindigo dye, a coumarin dye, an isoindolinone pigment, a diketopyrrolopyrrole pigment, a condensed azo pigment, a benzimidazolone pigment, a dioxazine pigment, a copper phthalocyanine pigment, and a quinacridone pigment.

These exemplified colorants may be used alone or in combination of two or more kinds thereof.

A content of the colorant in the composition of the present embodiment is, for example, 1.0 × 10⁻⁸ parts by mass to 0.5 parts by mass when the total amount of the composition is 100 parts by mass.

### (7) Ratio of Components

In the composition of the present embodiment, a ratio of the concentration of methyl propionate to the concentration of methyl isobutyrate (concentration of methyl propionate/concentration of methyl isobutyrate) is preferably 0.05 to 15.0, more preferably 0.08 to 8.0, and particularly preferably 0.1 to 2.5.

In the present embodiment, when the ratio of the concentration of methyl propionate to the concentration of methyl isobutyrate that can be contained in the composition is in such a range, the polymerization rate when the composition is polymerized can be further improved.

In the composition of the present embodiment, the ratio of the concentration of methyl acrylate to the concentration of methyl isobutyrate (concentration of methyl acrylate/concentration of methyl isobutyrate) described above is preferably 0.001 to 5,000 and more preferably 0.01 to 1,000.

In the present embodiment, when the ratio of the concentration of methyl acrylate to the concentration of methyl isobutyrate that can be contained in the composition is in such a range, the polymerization rate when the composition is polymerized can be further improved.

### 2. Method of Producing Composition

The composition of the present embodiment can be produced by mixing the components described above under any suitable conditions by any conventionally-known suitable method.

Specifically, the composition can be produced by preparing the above components in a predetermined amount and mixing the components in a mixing tank controlled at a constant temperature with a heat medium jacket or the like having any conventionally-known suitable configuration.

In this manner, according to the method of producing a composition of the present embodiment, it is possible to easily produce a composition that enables production of a polymer (molded body) having the excellent properties described above at low cost without large facilities.

### 3. Method of Producing Polymethyl Methacrylate (Polymer) and Molded Body Thereof

The composition of the present embodiment can be formed into a polymer by a method of forming a polymer including polymerizing the composition. A method of forming a composition into a polymer of the present embodiment is not particularly limited. Specifically, the composition can be polymerized into a polymer by any suitable method selected in consideration of components contained in the composition of the present embodiment and contents of the components.

Examples of the method of forming a composition into a polymer include bulk polymerization, solution polymerization, suspension polymerization, and emulsion polymerization.

Specifically, a sheet that is a molded body of polymethyl methacrylate can be obtained by polymerizing the composition of the present embodiment by, for example, a bulk polymerization method. In addition, in cell cast polymerization, any suitable heat treatment can be performed on the composition under predetermined heating conditions to advance polymerization reactions. Accordingly, the composition is polymerized and cured, thereby obtaining a polymer (molded body).

In the method of polymerizing the composition of the present embodiment (the production method of a molded body), a heating temperature and a heating time in the heating conditions can be set in consideration of, for example, the types and contents of the selected polymerization modifier, polymerization initiator, and/or other components, the content of the methyl methacrylate, the concentrations of the methyl isobutyrate, methyl propionate, and methyl acrylate, and the like.

In the present embodiment, in the cell cast polymerization, a heating temperature is set to, for example, 50°C to 120°C and a heating time can be set to, for example, 1 hour to 20 hours to produce a polymer and a molded body thereof.

The heat treatment in the method of producing a polymer and a molded body thereof of the present embodiment can involve a plurality of steps performed at different heating temperatures and/or heating times.

The polymer of the present embodiment and a molded body thereof can be produced by the heat treatment under heating conditions including, for example, the following Steps 1 to 7.
Step 1: Raise the temperature from room temperature to 68°C for 20 minutes.
Step 2: Hold the temperature at 68°C for 90 minutes.
Step 3: Lower the temperature from 68°C to 64°C for 20 minutes.
Step 4: Hold the temperature at 64°C for 90 minutes.
Step 5: Raise the temperature from 64°C to 123°C for 10 minutes.
Step 6: Hold the temperature at 123°C for 120 minutes.
Step 7: Lower the temperature from 123°C to room temperature for 78 minutes.

In the method of producing a polymer and a molded body thereof of the present embodiment, Steps 1 to 7 are performed in this order, such that it possible to suppress heat generation in the polymerization and to terminate the polymerization stably.

In the heat treatment of the composition of the present embodiment, for example, a cell casting method (cell cast polymerization) is applied using a cell that defines a predetermined-shaped sealed space inside the cell, thereby forming a molded body with a predetermined shape. Hereinafter, a method of producing a molded body by the cell casting method will be described in detail.

In producing a molded body by the cell casting method, first, a cell is prepared. Here, a plate-shaped molded body (may be referred to as "cast plate") will be described.

Such a cell can include at least two flat-plate members and a seal material (gasket) that is sandwiched between the two flat-plate members and can seal a gap between the two flat-plate members facing each other to form a sealed space.

The flat-plate member may have the form of a sheet or belt. The flat-plate member includes a material that is not dissolved by the composition of the present embodiment, does not inhibit polymerization reactions, and has sufficient heat resistance to the heating temperature during the heat treatment. Preferred examples of the material of the flat-plate member include glass and a metal.

As the seal material, any conventionally-known suitable seal material may be used. The seal material includes a material that is not dissolved by the composition of the present embodiment, does not inhibit polymerization reactions, and has sufficient heat resistance to the heating temperature during the heat treatment. Specific preferred examples of the seal material include a vinyl chloride resin gasket.

Next, the composition of the present embodiment is injected into a gap (void) defined by the cell prepared as described above by any conventionally-known suitable method.

Next, the cell is subjected to heat treatment under the above heating conditions. The heat treatment for the cell to which the composition of the present embodiment is injected is not particularly limited. Similarly to the conventionally-known cell casting method, the heat treatment method for the cell can be a method of directly performing heat treatment from the outside of the cell using, for example, a hot air circulation furnace or an infrared heater, and a method of further providing any conventionally-known suitable jacket outside the cell and introducing a heat medium such as hot air, hot water, and water vapor into the jacket.

### 4. Measurement of Residual Amount of Methyl Methacrylate and Calculation of Polymerization Rate of Methyl Methacrylate

In the present embodiment, measurement of a residual amount of the methyl methacrylate can be performed by any conventionally-known suitable method using any conventionally-known suitable gas chromatography apparatus using a molded body containing polymerized polymethyl methacrylate (for example, the cast plate described above). Specific examples thereof are as follows.

First, for example, a sample obtained by cutting the molded body produced as described above is precisely weighed so as to be a predetermined amount, and a solvent such as acetone is added and dissolved to obtain a solution.

Next, an internal standard solution (for example, a solution obtained by dissolving 1% of methyl isobutyl ketone (MIBK) in methanol) is added to the obtained solution, and the mixture is stirred to obtain a mixed solution.

Next, methanol or the like is added to the obtained mixed solution to reprecipitate a methyl methacrylate polymer, and then a supernatant solution is used as a sample solution.

Next, the obtained sample solution is measured under predetermined conditions using any conventionally-known suitable gas chromatography apparatus (for example, GC-2010 Plus (manufactured by Shimadzu Corporation), column: DB-1 (manufactured by Agilent Technologies, Inc.), detector: FID 2010 Plus (manufactured by Shimadzu Corporation)).

Then, a peak area (a1) corresponding to methyl methacrylate and a peak area (b1) corresponding to methyl isobutyl ketone, which are detected when the sample solution is measured, are measured.

Next, a peak area ratio A (= a1/b1) is obtained from the obtained peak area.

Specifically, a standard in which a mass ratio of the content of the methyl methacrylate to the content of the methyl isobutyl ketone is W0 (known) is measured in the same manner as described above, and a peak area (a0) corresponding to the detected methyl methacrylate and a peak area (b0) corresponding to the methyl isobutyl ketone are measured.

Next, a peak area ratio A0 (= a0/b0) is obtained from the obtained peak area. A factor f (= W0/A0) is obtained from the obtained peak area ratio A0 and the mass ratio W0.

Next, a mass ratio W of the methyl methacrylate to the methyl isobutyl ketone contained in the sample solution is calculated by multiplying the peak area ratio A by the factor **f.** A residual amount (% by mass) of the methyl methacrylate contained in the molded body is calculated from the calculated mass ratio W and the weight of the molded body used for preparing the sample solution. As described above, the residual amount (% by mass) of the methyl methacrylate can be calculated.

When the residual amount of the methyl methacrylate present in the molded body is large, the mechanical properties and heat resistance properties of the molded body tend to be deteriorated, and therefore, the residual amount of the methyl methacrylate is preferably small. The residual amount of the methyl methacrylate calculated by the above method is preferably less than 0.690% by mass and more preferably 0.680% by mass or less.

In the calculation of the polymerization rate of the methyl methacrylate, the polymerization rate (%) of the methyl methacrylate can be calculated by the following formula using the residual amount (% by mass) of the methyl methacrylate calculated as described above. (polymerization rate of methyl methacrylate) = ((content of methyl methacrylate in composition - residual amount of methyl methacrylate in molded body)/content of methyl methacrylate in composition) × 100

When the polymerization rate of the methyl methacrylate is high, the amount of polymerization per unit time when the composition is polymerized increases, such that the amount of the obtained polymer increases. As the production scale increases, the influence of the polymerization rate of the methyl methacrylate on the production efficiency of the polymer increases. In addition, when the polymerization rate of the methyl methacrylate is high, the energy efficiency in the production of the polymer also increases, and thus, the load on the environment can also be reduced. The polymerization rate of the methyl methacrylate is preferably more than 99.30% and more preferably 99.32% or more.

### 5. Use of Polymer and Molded Body Thereof

A polymer of the composition of the present embodiment and a molded body thereof are excellent in light permeability, heat resistance, and weather resistance. Therefore, the present invention can be suitably applied to various applications such as lighting equipment, automobile parts, signboards, and building materials that may be exposed to external environments and also to heat sources and light sources.

### EXAMPLES

Examples according to embodiments of the present invention will be described. The present invention is not limited by the following Examples.

### <Preparation Example 1>

### . Preparation of Methyl Methacrylate Product

Methacrylic acid and methanol were esterified inside a reactor to synthesize methyl methacrylate, and then, unreacted methanol was removed in an extraction column. Purification was repeated in a plurality of distillation columns, thereby preparing a methyl methacrylate product containing extremely high-purity methyl methacrylate.

Methyl isobutyrate (10.9 ppm by mass) and methyl acrylate (1.1 ppm by mass) were contained in the obtained methyl methacrylate product. A content of the methyl methacrylate in the obtained methyl methacrylate product was 99.99% by mass.

### <Example 1>

### · Preparation of Composition 1

Methyl isobutyrate (manufactured by FUJIFILM Wako Pure Chemical Corporation) and methyl propionate (manufactured by FUJIFILM Wako Pure Chemical Corporation) were added to and mixed with the methyl methacrylate product prepared in Preparation Example 1, thereby preparing Composition 1. Composition 1 obtained was in the form of a solution.

A content of the methyl methacrylate in Composition 1 obtained was 99.88% by mass. A concentration of the methyl isobutyrate in Composition 1 was 1,000 ppm by mass, a concentration of the methyl acrylate in Composition 1 was 1.1 ppm by mass, and a concentration of the methyl propionate in Composition 1 was 200 ppm by mass. Table 1 shows the composition of Composition 1.

### · Preparation of Composition 1'

Composition 1 (99.83 parts by mass) prepared as described above, sodium di-(2-ethylhexyl) sulfosuccinate (0.05 parts by mass) as a release agent, terpinolene (0.01 parts by mass) as a polymerization modifier, 2,2'-azobisisobutyronitrile (0.08 parts by mass) as a polymerization initiator, and 2-(2'-hydroxy-5'-methylphenyl)benzotriazole (0.01 parts by mass) as an ultraviolet absorber were added to a glass container and mixed to obtain Composition 1' for forming a molded body (cast plate). Composition 1' obtained was in the form of a solution.

### · Preparation of Cast Plate 1

A vinyl chloride resin gasket having a thickness of 3.8 mm was sandwiched between two glass plates facing each other so as to prepare a cell defining a gap sealed by the vinyl chloride resin gasket and the two glass plates. Composition 1' prepared as described above was injected into the gap inside the cell. The cell into which Composition 1' was injected was placed in an oven and subjected to heat treatment under heating conditions including the following Steps 1 to 7 in this order to polymerize methyl methacrylate, thereby preparing Cast plate 1, which is a 250 mm-square molded body having a thickness of 3 mm.

### (Heating Condition)

Step 1: Raise the temperature from room temperature to 68°C for 20 minutes.
Step 2: Hold the temperature at 68°C for 90 minutes.
Step 3: Lower the temperature from 68°C to 64°C for 20 minutes.
Step 4: Hold the temperature at 64°C for 90 minutes.
Step 5: Raise the temperature from 64°C to 123°C for 10 minutes.
Step 6: Hold the temperature at 123°C for 120 minutes.
Step 7: Lower the temperature from 123°C to room temperature for 90 minutes.

### · Measurement of Residual Amount of Methyl Methacrylate Contained in Cast Plate 1

### (Preparation of Sample Solution 1)

0.5 g of Cast Plate 1 prepared as described above was cut and precisely weighed, and 10 cc of acetone (special grade) was added and dissolved. 1 cc of an internal standard solution (a solution obtained by dissolving 1% of methyl isobutyl ketone (MIBK) in methanol) was added to the obtained acetone solution, and stirring was performed. 30 cc of methanol was added to the obtained mixed solution to reprecipitate a methyl methacrylate polymer, and then a supernatant solution was collected as Sample Solution 1.

### · Measurement of Residual Amount of Methyl Methacrylate

Sample Solution 1 was measured under the following conditions using the following gas chromatography apparatus.

### (Measurement Conditions)

Apparatus: GC-2010 Plus (manufactured by Shimadzu Corporation)
Column: DB-1 (manufactured by Agilent Technologies, Inc.)
Detector: FID 2010 Plus (manufactured by Shimadzu Corporation)
Column oven conditions
Initial temperature: 40°C (hold time: 1 minute)
Heating rate: 8 °C/min
Intermediate temperature: 120°C (hold time: 0 minutes)
Heating rate: 20 °C/min
Final temperature: 250°C (hold time: 5 minutes)
Sample vaporization conditions
Vaporizing chamber temperature: 300°C
Carrier gas: helium
Pressure: 50 kPa
Total flow rate: 58.3 mL/min
Column flow rate: 1.08 mL/min
Linear velocity: 31.1 cm/sec
Purge dose: 3.0 mL/min
Split ratio: 50
Detector conditions
Detector temperature: 300°C
Sampling rate: 40 msec
Make-up gas: N₂
Make-up flow rate: 30 mL/min
H2 flow rate: 40 mL/min
Air flow rate: 400 mL/min
Autosampler conditions
Injection amount: 1 µL

A peak area (a1) corresponding to methyl methacrylate and a peak area (b1) corresponding to methyl isobutyl ketone, which were detected when Sample Solution 1 was measured under the measurement conditions described above, were measured. Then, a peak area ratio A (= a1/b1) was obtained from these obtained peak areas.

A standard in which a mass ratio of the content of the methyl methacrylate to the content of the methyl isobutyl ketone was W0 (known) was measured under the measurement conditions described above, and a peak area (a0) corresponding to the detected methyl methacrylate and a peak area (b0) corresponding to the methyl isobutyl ketone were measured. Then, a peak area ratio A0 (= a0/b0) was obtained from these obtained peak areas.

Next, a factor f (= W0/A0) was obtained from the obtained peak area ratio A0 and the mass ratio W0.

Next, a mass ratio W of the methyl methacrylate to the methyl isobutyl ketone contained in Sample Solution 1 was calculated by multiplying the peak area ratio A by the factor f. A residual amount (% by mass) of the methyl methacrylate contained in Cast Plate 1 (methyl methacrylate residual amount) was calculated from the calculated mass ratio W and the weight of Cast Plate 1 used for preparing Sample Solution 1. The results are shown in Table 2.

### · Calculation of Polymerization Rate of Methyl Methacrylate

The polymerization rate (%) of the methyl methacrylate (methyl methacrylate polymerization rate) was calculated by the following formula using the residual amount of the methyl methacrylate calculated. The results are shown in Table 2. (polymerization rate of methyl methacrylate) = ((content of methyl methacrylate in Composition 1' - residual amount of methyl methacrylate in Cast Plate 1)/content of methyl methacrylate in Composition 1') × 100

### <Example 2>

### · Preparation of Composition 2

Methyl isobutyrate and methyl propionate were added to and mixed with the methyl methacrylate product prepared in Preparation Example 1, thereby preparing Composition 2. Composition 2 obtained was in the form of a solution.

A content of the methyl methacrylate in Composition 2 was 99.85% by mass. A concentration of the methyl isobutyrate in Composition 2 was 1,000 ppm by mass, a concentration of the methyl acrylate in Composition 1 was 1.1 ppm by mass, and a concentration of the methyl propionate in Composition 2 was 501 ppm by mass. Table 1 shows the composition of Composition 2.

Composition 2' was prepared in the same manner as that of Example 1 except for using Composition 2 prepared as described above, Cast Plate 2 was prepared, and the residual amount of the methyl methacrylate and the polymerization rate of the methyl methacrylate were measured. The results are shown in Table 2.

### <Example 3>

### · Preparation of Composition 3

Methyl isobutyrate, methyl acrylate, and methyl propionate were further added to and mixed with the methyl methacrylate product prepared in Preparation Example 1, thereby preparing Composition 3. Composition 3 obtained was in the form of a solution.

A content of the methyl methacrylate in Composition 3 obtained was 99.94% by mass. A concentration of the methyl isobutyrate in Composition 3 was 40.9 ppm by mass, a concentration of the methyl acrylate in Composition 3 was 51.1 ppm by mass, and a concentration of the methyl propionate in Composition 3 was 500 ppm by mass. Table 1 shows the composition of Composition 3.

Composition 3' was prepared in the same manner as that of Example 1 except for using Composition 3 prepared as described above, Cast Plate 3 was prepared, and the residual amount of the methyl methacrylate and the polymerization rate of the methyl methacrylate were measured. The results are shown in Table 2.

### <Example 4>

### · Preparation of Composition 4

Methyl isobutyrate and methyl propionate were added to and mixed with the methyl methacrylate product prepared in Preparation Example 1, thereby preparing Composition 4. Composition 4 obtained was in the form of a solution.

A content of the methyl methacrylate in Composition 4 obtained was 99.80% by mass. A concentration of the methyl isobutyrate in Composition 4 was 1,005 ppm by mass, a concentration of the methyl acrylate in Composition 4 was 1.1 ppm by mass, and a concentration of the methyl propionate in Composition 4 was 1,005 ppm by mass. Table 1 shows the composition of Composition 4.

Composition 4' was prepared in the same manner as that of Example 1 except for using Composition 4 prepared as described above, Cast Plate 4 was prepared, and the residual amount of the methyl methacrylate and the polymerization rate of the methyl methacrylate were measured. The results are shown in Table 2.

### <Example 5>

### · Preparation of Composition 5

Methyl isobutyrate and methyl propionate were added to and mixed with the methyl methacrylate product prepared in Preparation Example 1, thereby preparing Composition 5. Composition 5 obtained was in the form of a solution.

A content of the methyl methacrylate in Composition 5 obtained was 99.70% by mass. A concentration of the methyl isobutyrate in Composition 5 was 1,004 ppm by mass, a concentration of the methyl acrylate in Composition 5 was 1.1 ppm by mass, and a concentration of the methyl propionate in Composition 5 was 2,007 ppm by mass. Table 1 shows the composition of Composition 5.

Composition 5' was prepared in the same manner as that of Example 1 except for using Composition 5 prepared as described above, Cast Plate 5 was prepared, and the residual amount of the methyl methacrylate and the polymerization rate of the methyl methacrylate were measured. The results are shown in Table 2.

### <Comparative Example 1>

### · Preparation of Composition C1

Methyl isobutyrate, methyl acrylate, and methyl propionate were added to and mixed with the methyl methacrylate product prepared in Preparation Example 1, thereby preparing Composition C1. Composition C1 obtained was in the form of a solution.

A content of the methyl methacrylate in Composition C1 obtained was 99.98% by mass. A concentration of the methyl isobutyrate in Composition C1 was 40.9 ppm by mass, a concentration of the methyl acrylate in Composition C1 was 51.1 ppm by mass, and a concentration of the methyl propionate in Composition C1 was 101 ppm by mass. Table 1 shows the composition of Composition C1.

Composition C1' was prepared in the same manner as that of Example 1 except for using Composition C1 prepared as described above, Cast Plate C1 was prepared, and the residual amount of the methyl methacrylate and the polymerization rate of the methyl methacrylate were measured. The results are shown in Table 2.

### <Comparative Example 2>

### · Preparation of Composition C2

Methyl isobutyrate, methyl acrylate, and methyl propionate were added to and mixed with the methyl methacrylate product prepared in Preparation Example 1, thereby preparing Composition C2. Composition C2 obtained was in the form of a solution.

A content of the methyl methacrylate in Composition C2 obtained was 99.47% by mass. A concentration of the methyl isobutyrate in Composition C2 was 30 ppm by mass, a concentration of the methyl acrylate in Composition C2 was 1.1 ppm by mass, and a concentration of the methyl propionate in Composition C2 was 5,200 ppm by mass. Table 3 shows the composition of Composition C2.

Composition C2' was prepared in the same manner as that of Example 1 except for using Composition C2 prepared as described above, Cast Plate C2 was prepared, and the residual amount of the methyl methacrylate and the polymerization rate of the methyl methacrylate were measured. Table 4 shows the results.

### <Comparative Example 3>

### · Preparation of Composition C3

Methyl isobutyrate, methyl acrylate, and methyl propionate were added to and mixed with the methyl methacrylate product prepared in Preparation Example 1, thereby preparing Composition C3. Composition C3 obtained was in the form of a solution.

A content of the methyl methacrylate in Composition C3 obtained was 99.48% by mass. A concentration of the methyl isobutyrate in Composition C3 was 5,200 ppm by mass, a concentration of the methyl acrylate in Composition C3 was 1.1 ppm by mass, and a concentration of the methyl propionate in Composition C3 was 52 ppm by mass. Table 3 shows the composition of Composition C3.

Composition C3' was prepared in the same manner as that of Example 1 except for using Composition C3 prepared as described above, Cast Plate C3 was prepared, and the residual amount of the methyl methacrylate and the polymerization rate of the methyl methacrylate were measured. Table 4 shows the results.

### <Comparative Example 4>

### · Preparation of Composition C4

Methyl isobutyrate, methyl acrylate, and methyl propionate were added to and mixed with the methyl methacrylate product prepared in Preparation Example 1, thereby preparing Composition C4. Composition C4 obtained was in the form of a solution.

A content of the methyl methacrylate in Composition C4 obtained was 98.98% by mass. A concentration of the methyl isobutyrate in Composition C4 was 5,200 ppm by mass, a concentration of the methyl acrylate in Composition C4 was 1.1 ppm by mass, and a concentration of the methyl propionate in Composition C4 was 5,000 ppm by mass. Table 3 shows the composition of Composition C4.

Composition C4' was prepared in the same manner as that of Example 1 except for using Composition C4 prepared as described above, Cast Plate C4 was prepared, and the residual amount of the methyl methacrylate and the polymerization rate of the methyl methacrylate were measured. Table 4 shows the results.

### [Table 1]

**(Table 1)**

| | Components | | | | Methyl |
|---|---|---|---|---|---|
| | Methyl methacrylate | Methyl isobutyrate | Methyl acrylate | Methyl propionate | propionate/methyl isobutyrate |
| Example 1 (Composition 1) | 99.88% by mass | 1,000 ppm by mass | 1.1 ppm by mass | 200 ppm by mass | 0.2 |
| Example 2 (Composition 2) | 99.85% by mass | 1,000 ppm by mass | 1.1 ppm by mass | 501 ppm by mass | 0.5 |
| Example 3 (Composition 3) | 99.94% by mass | 41 ppm by mass | 51 ppm by mass | 500 ppm by mass | 12.2 |
| Example 4 (Composition 4) | 99.80% by mass | 1,005 ppm by mass | 1.1 ppm by mass | 1,005 ppm by mass | 1.0 |
| Example 5 (Composition 5) | 99.70% by mass | 1,004 ppm by mass | 1.1 ppm by mass | 2,007 ppm by mass | 2.0 |
| Comparative Example 1 (Composition C1) | 99.98% by mass | 41 ppm by mass | 51 ppm by mass | 101 ppm by mass | 2.5 |

### [Table 2]

**(Table 2)**

| | Methyl methacrylate residual amount | Methyl methacrylate polymerization rate |
|---|---|---|
| Example 1 (Cast Plate 1) | 0.663% by mass | 99.34% |
| Example 2 (Cast Plate 2) | 0.678% by mass | 99.32% |
| Example 3 (Cast Plate 3) | 0.672% by mass | 99.33% |
| Example 4 (Cast Plate 4) | 0.657% by mass | 99.34% |
| Example 5 (Cast Plate 5) | 0.626% by mass | 99.37% |
| Comparative Example 1 (Cast Plate C1) | 0.699% by mass | 99.30% |

### [Table 3]

**(Table 3)**

| | Components | | | | Methyl |
|---|---|---|---|---|---|
| | Methyl methacrylate | Methyl isobutyrate | Methyl acrylate | Methyl propionate | propionate/methyl isobutyrate |
| Comparative Example 2 (Composition C2) | 99.47% by mass | 30 ppm by mass | 1.1 ppm by mass | 5,200 ppm by mass | 173.3 |
| Comparative Example 3 (Composition C3) | 99.48% by mass | 5,200 ppm by mass | 1.1 ppm by mass | 52 ppm by mass | 0.01 |
| Comparative Example 4 (Composition C4) | 99.98% by mass | 5,200 ppm by mass | 1.1 ppm by mass | 5,000 ppm by mass | 1.0 |

### [Table 4]

**(Table 4)**

| | Methyl methacrylate residual amount | Methyl methacrylate polymerization rate |
|---|---|---|
| Comparative Example 2 (Cast Plate C2) | 0.786% by mass | 99.21% |
| Comparative Example 3 (Cast Plate C3) | 0.772% by mass | 99.22% |
| Comparative Example 4 (Cast Plate C4) | 0.733% by mass | 99.26% |

## Claims

1. A composition comprising methyl methacrylate, methyl isobutyrate, and methyl propionate,
wherein a content of the methyl methacrylate is 90% by mass or more, and
at least one requirement that is selected from a group consisting of the following requirements (1) and (2) is satisfied,
Requirement (1): a concentration of the methyl isobutyrate is from 20 ppm by mass to 3,000 ppm by mass, and a concentration of the methyl propionate is from 250 ppm by mass to 3,000 ppm by mass,
Requirement (2): a concentration of the methyl isobutyrate is from 350 ppm by mass to 3,000 ppm by mass, and a concentration of the methyl propionate is from 5 ppm by mass to 250 ppm by mass.

2. The composition according to claim 1, wherein the content of the methyl methacrylate is 95% by mass or more.

3. The composition according to claim 2, wherein the content of the methyl methacrylate is 98% by mass or more.

4. The composition according to any one of claims 1 to 3, wherein the requirement (1) is satisfied.

5. The composition according to any one of claims 1 to 3, wherein the concentration of the methyl isobutyrate is from 20 ppm by mass to 2,500 ppm by mass.

6. The composition according to any one of claims 1 to 3, wherein the concentration of the methyl propionate is 250 ppm by mass to 2,500 ppm by mass.

7. The composition according to any one of claims 1 to 3, wherein a ratio of the concentration of the methyl propionate to the concentration of the methyl isobutyrate is from 0.1 to 2.5.

8. The composition according to any one of claims 1 to 3, further comprising
methyl acrylate,
wherein a concentration of the methyl acrylate is from 5 ppm by mass to 50,000 ppm by mass.

9. The composition according to any one of claims 1 to 3, wherein
one or two or more selected from a group consisting of methyl methacrylate, methyl isobutyrate, and methyl propionate include a component regenerated from polymethyl methacrylate.

10. The composition according to any one of claims 1 to 3, further comprising
one or two or more selected from a group consisting of methanol, methyl 2,4-dimethyl-4-pentenoate, 1-butanol, and butyl acrylate,
wherein a concentration of each of the methanol, methyl 2,4-dimethyl-4-pentenoate, 1-butanol, and butyl acrylate is from 10 ppm by mass to 5,000 ppm by mass.

11. The composition according to any one of claims 1 to 3, further comprising
one or two or more selected from a group consisting of methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene,
wherein a concentration of each of the methyl 2-methyl-3-butenoate, methyl tiglate, methyl 3-methyl-3-butenoate, methyl 3-methyl-2-butenoate, dimethyl itaconate, dimethyl 2-methyl-5-methylenehexanedioate, toluene, and styrene is from 10 ppm by mass to 1,000 ppm by mass.

12. A method of producing a polymer, the method comprising polymerizing the composition according to any one of claims 1 to 3.
